# EUROPÄISCHE PATENTSCHRIFT

(11) **EP 3 771 445 B1**
(45) Veröffentlichungstag und Bekanntmachung des Hinweises auf die Patenterteilung: **06.03.2024**
(21) Anmeldenummer: 20184672.2
(22) Anmeldetag: 08.07.2020
(51) Int. Cl.: A61B 17/32, A61B 90/00

(54) **CHIRURGISCHES INSTRUMENT**
SURGICAL INSTRUMENT
INSTRUMENT CHIRURGICAL

(30) Priorität: 31.07.2019 DE 102019120671
(43) Veröffentlichungstag der Anmeldung: 03.02.2021
(73) Patentinhaber: Pajunk GmbH Medizintechnologie, 78187 Geisingen (DE)
(72) Erfinder: PAJUNK-SCHELLING, Simone, 78187 Geisingen (DE); HAUGER, Martin, 78166 Donaueschingen (DE)
(74) Vertreter: Westphal, Mussgnug & Partner Patentanwälte mbB

(56) Entgegenhaltungen:
- EP-B1- 3 193 748
- WO-A1-94/26182
- US-A1- 2014 121 456
- US-A1- 2014 171 790
- US-A1- 2017 042 565
- US-A1- 2019 110 851
- US-B1- 6 358 211

## Beschreibung

Die Erfindung betrifft ein chirurgisches Instrument.

Der Mittelnerv der Hand, der die Muskeln des Daumens und der Mittelhand versorgt, verläuft durch eine tunnelartige Röhre, dem sogenannten Karpaltunnel. In Höhe des Handgelenkes spannt sich zwischen Daumen- und Kleinfingermuskulatur ein breites Band aus. Dieses Band, welches auch als Retinaculum flexorum bezeichnet wird, ist das Dach des Karpaltunnels, der den Mittelhandnerven und die Beugesehnen der Finger enthält. Beim Karpaltunnelsyndrom wird dieser Kanal zu eng und es entsteht Druck auf den Mittelhandnerven. Dies kann zu Beschwerden beispielsweise in Form von kribbelnden oder eingeschlafenen Händen führen.

In einigen Fällen ist zur Behandlung des Karpaltunnelsyndroms ein operativer Eingriff erforderlich. Bei der Operation wird der Nervenkanal erweitert, indem das Retinaculum flexorum, welches die Handwurzelknochen überspannt und den Karpaltunnel hohlhandwärts begrenzt, durchtrennt wird. Dieser Eingriff ist sowohl offen als auch minimalinvasiv durchführbar. Bei einem möglichen Verfahren zur minimalinvasiven Karpaldachspaltung wird ein ca. zwei Zentimeter langer Schnitt im Bereich des beugeseitigen Handgelenkes gemacht, durch welchen eine kleine Sonde mit einem kleinen Messer in den Karpaltunnel, zwischen dem Mittelhandnerven und dem Karpaldach, eingeführt. Wenn sich die Sonde im Karpaltunnel befindet, kann das Karpaldach von innen gespalten werden. Bei einer alternativen Operationsmethode wird an einem Ende des Karpaltunnels ein 2 bis 3 cm langer Schnitt gemacht und der dann sichtbare Teil des queren Handgelenksbandes unter direkter Sicht durchtrennt. Der restliche Teil des Bandes kann entweder mit einer Schere oder einem speziellen Messer ohne direkte Sicht, also blind, durchgeschnitten werden.

Für den minimalinvasiven Eingriff ist ein chirurgisches Instrument mit einem Handgriff und einer daran angeordneten Klinge mit einer konkav ausgebildeten Schneidkante bekannt, wobei die Klinge eine Ebene aufweist. Der minimalinvasive Eingriff weist allerdings den Nachteil auf, dass die Klinge in kurzer Entfernung zu den in der Hand verlaufenden Nerven vorgeschoben werden muss, so dass die Gefahr einer Beschädigung der Nerven besteht.

Ein gattungsgemäßes chirurgisches Instrument offenbart die WO 94/26182 A1. Als weiterer Stand der Technik werden die US 2014/121456 A1, die US 2017/042565 A1 sowie die US 2014/171790 A1 genannt.

Die Aufgabe der Erfindung besteht daher darin, ein chirurgisches Instrument bereit zu stellen, mit welchem die Sicherheit des minimalinvasiven Eingriffs am Karpaltunnel einer Hand erhöht werden kann.

Die Aufgabe der Erfindung wird gelöst durch ein chirurgisches Instrument mit den Merkmalen des Patentanspruchs 1.

Vorteilhafte Ausgestaltungen und Weiterbildungen der Erfindung sind in den abhängigen Ansprüchen angegeben.

Das erfindungsgemäße chirurgische Instrument mit einem Handgriff und einer daran angeordneten Klinge mit einer konkav ausgebildeten Schneidkante, wobei die Klinge eine Ebene aufweist, zeichnet sich dadurch aus, dass das chirurgische Instrument ultraschallsichtbarkeitserhöhende Mittel aufweist.

Durch die Anordnung von Mitteln, welche die Ultraschallsichtbarkeit erhöhen, wird das chirurgische Instrument bei dem minimalinvasiven Eingriff sichtbar, so dass der Chirurg zu jedem Zeitpunkt erkennen kann, an welcher Position sich die Klinge des chirurgischen Instruments in der Hand befindet und ob die Gefahr besteht, einen Nerv zu verletzen. Dadurch wird es ermöglicht, rechtzeitig die Vorschubbewegung des chirurgischen Instruments zu korrigieren und Nervverletzungen zu vermeiden.

Erfindungsgemäß umfassen die Mittel Ultraschallreflektoren, welche eine besonders gute Erhöhung der Ultraschallsichtbarkeit ermöglichen.

Gemäß der Erfindung sind die Ultraschallreflektoren als Vertiefungen ausgebildet. Dies ermöglicht eine einfache Herstellung der ultraschallsichtbarkeitserhöhenden Mittel.

Weiterhin sieht die Erfindung vor, dass die Vertiefungen jeweils mindestens zwei, vorzugsweise genau drei jeweils senkrecht zueinander ausgerichtete, Seitenflächen aufweisen. Derartige Vertiefungen ermöglichen eine besonders gute Ultraschallsichtbarkeit. Insbesondere Vertiefungen mit genau drei jeweils senkrecht zueinander ausgerichteten Seitenflächen ermöglichen eine gute Ultraschallsichtbarkeit aus unterschiedlichen Richtungen.

Gemäß einer vorteilhaften Weiterbildung der Erfindung sind die Mittel unlösbar an der Klinge angeordnet, beispielsweise angeklebt oder einstückig mit dieser verbunden. Bei einer derartigen Anordnung ist die relative Ausrichtung zwischen den Mitteln und der Klinge fixiert.

Eine bevorzugte Ausgestaltung der Erfindung sieht vor, dass die Mittel zumindest abschnittsweise mit einer Beschichtung versehen sind. Die Beschichtung ist insbesondere transparent für Ultraschall ausgestaltet. Eine derartige Beschichtung kann die Reinigbarkeit des chirurgischen Instruments verbessern, da beispielsweise die Vertiefungen mit der Beschichtung ausgefüllt sein können und die Beschichtung eine glatte Außenseite aufweisen kann.

Vorzugsweise sind die Mittel mit der Klinge zumindest abschnittsweise vergossen. Dadurch können Spalte oder Ritzen, in denen sich Verschmutzungen festsetzen können, verringert oder vermieden werden.

Erfindungsgemäß weisen die Mittel ein blattartiges Basiselement auf, welches eine Ebene aufweist. Ein derartiges Basiselement ermöglicht eine gezielte Ausrichtung der ultraschallsichtbarkeitsverstärkenden Mittel.

Gemäß der Erfindung sind die Ultraschallreflektoren auf dem Basiselement angeordnet. Dies ermöglicht eine einfache Herstellung.

Weiterhin sieht die Erfindung vor, dass die Ebene des blattartigen Basiselements im Wesentlichen senkrecht zu der Ebene der Klinge angeordnet ist. Dies ermöglicht eine besonders gute Ultraschallsichtbarkeit der Klinge, welche bei der Operation üblicherweise senkrecht zur Hautoberfläche angeordnet ist.

Die Ultraschallsichtbarkeit wird dann weiter verbessert, wenn vorzugsweise eine erste der Seitenflächen einer der Vertiefungen mit der Ebene des Basiselements einen Winkel von etwa 35° einschließt.

Eine weitere Verbesserung der Ultraschallsichtbarkeit kann erreicht werden, wenn vorteilhafterweise eine zweite und eine dritte der Seitenflächen symmetrisch zu einer Längsachse des Basiselements angeordnet sind.

Vorzugsweise weist die Vertiefungen eine dreieckige Grundfläche in der Außenfläche des Basiselements auf, wobei die Spitze des Dreiecks zu einem distalen Ende des chirurgischen Instruments weist. Eine derartige Ausgestaltung kann die Ultraschallsichtbarkeit weiter verbessern.

Gemäß einer besonders bevorzugten Ausführungsform der Erfindung ist das chirurgische Instrument stiftartig mit einem distalen und einem proximalen Ende ausgebildet, wobei die Klinge an dem distalen Ende, vorzugsweise stirnseitig am distalen Ende, angeordnet ist. Eine derartige Ausgestaltung ist günstig für einen guten minimalinvasiven Eingriff.

Vorzugsweise ist die Klinge an einem Klingenträger angeordnet ist.

Gemäß einer vorteilhaften Weiterbildung der Erfindung können die Mittel statt an der Klinge auch unlösbar an dem Klingenträger angeordnet sein, beispielsweise angeklebt oder einstückig mit diesem verbunden. Ist die Klinge in dem Klingenträger fixiert, kann auch auf diese Weise die relative Anordnung zwischen Klinge und Mitteln sichergestellt werden.

Der Klingenträger kann einstückig an dem Handgriff angeordnet sein. Eine besonders vorteilhafte Ausgestaltung der Erfindung sieht vor, dass die Klinge oder der Klingenträger lösbar an dem Handgriff befestigt ist, vorzugsweise mittels einer Schraubverbindung oder einer Bajonettverbindung. Eine lösbare Befestigung ermöglicht ein Auswechseln. Insbesondere wird es dadurch ermöglicht, die Klinge oder den Klingenträger einschließlich der Klinge als Einwegprodukt auszugestalten, welches nach Gebrauch entsorgt werden kann, während der Handgriff als Mehrwegprodukt ausgestaltet sein kann.

Vorteilhafterweise weist das chirurgische Instrument einen ersten Abschnitt, welcher vorzugsweise im Wesentlichen von dem Handgriff gebildet ist, mit einer ersten Längsachse und einen zweiten Abschnitt, welcher vorzugsweise im Wesentlichen von dem Klingenträger gebildet ist, mit einer zweiten Längsachse auf, wobei die zweite Längsachse um einen Winkel gegen die erste Längsachse abgewinkelt ist, welcher im Bereich von 10° bis 40°, vorzugsweise im Bereich von 20° bis 30°, liegt und besonders bevorzugt 25° beträgt. Eine derartige Ausgestaltung ist günstig für einen guten minimalinvasiven Eingriff.

Vorzugsweise liegt der Winkel, um den die zweite Längsachse gegen die erste Längsachse abgewinkelt ist, in der Ebene der Klinge, wodurch die Handhabung bei dem operativen Eingriff vereinfacht werden kann.

Ein Ausführungsbeispiel der Erfindung wird anhand der nachfolgenden Figuren ausführlich erläutert. Es zeigen
- Figur 1: eine perspektivische Ansicht eines Ausführungsbeispiels eines erfindungsgemäßen chirurgischen Instruments,
- Figur 2: eine Ausschnittvergrößerung des distalen Endes des chirurgischen Instruments gemäß Figur 1,
- Figur 3: das distale Ende des chirurgischen Instruments wie in Figur 2 dargestellt mit ausgeblendetem Klingenhalter,
- Figur 4: eine Draufsicht auf das distale Ende gemäß Figur 3,
- Figur 5: eine Ansicht von vorn auf das distale Ende gemäß Figur 3,
- Figur 6: eine Draufsicht auf das chirurgische Instrument gemäß Figur 1,
- Figur 7: einen Schnitt entlang der Linie A-A in Figur 6 und
- Figur 8: eine Ausschnittvergrößerung aus Figur 3.

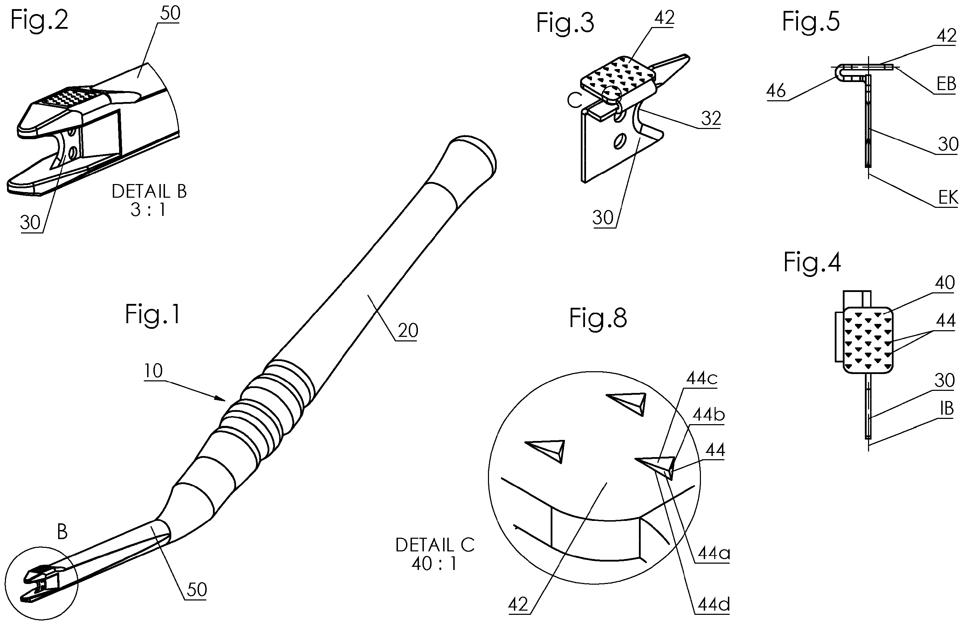

Die Figuren 1 bis 8 zeigen verschiedene Ansichten eines Ausführungsbeispiels eines erfindungsgemäßen chirurgischen Instruments 10.

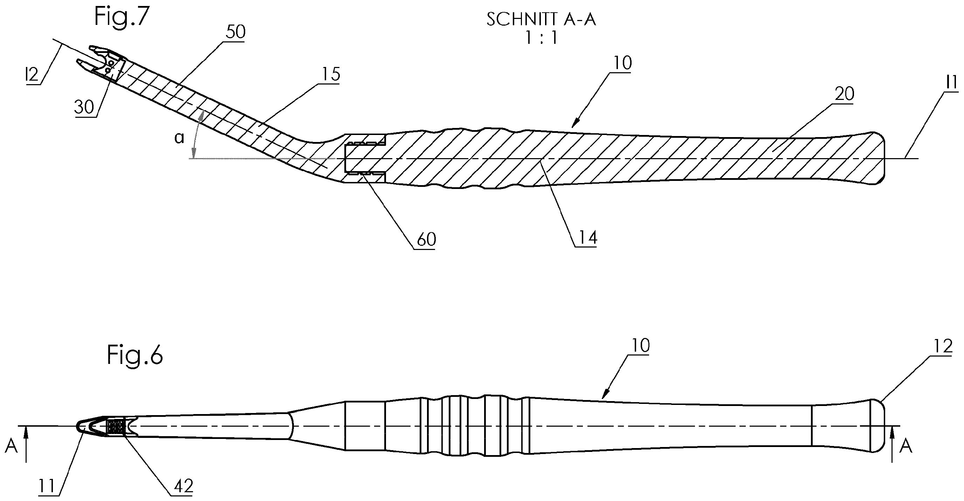

Das Instrument 10 weist einen Handgriff 20 und eine daran angeordnete Klinge 30 mit einer konkav ausgebildeten Schneidkante 32 auf. Die Klinge 30 kann blattartig ausgebildet sein und weist dabei eine Ebene EK auf, welche parallel zu einer Oberfläche verläuft. Die Ebene EK der Klinge 30 ist in Figur 7 die Papierebene und ist in Figur 5 im Schnitt erkennbar. Die Dicke der blattartigen Klinge sollte ausreichend sein, um genügend Stabilität zu gewährleisten. Die Schneidkante 32 kann beispielsweise U-förmig oder V-förmig ausgebildet sein. Die Schneidkante 32 liegt insbesondere in der Ebene EK der Klinge 30. Die Schneidkante 32 ist beispielsweise ausgehend von einer der Seitenkanten der blattartigen Klinge 30 als konkave Ausnehmung ausgebildet.

Die Klinge 30 kann direkt an dem Handgriff 20 angeordnet sein. Im vorliegenden Ausführungsbeispiel ist die Klinge 30 in einem Klingenträger 50 befestigt, welcher an dem Handgriff 20 angeordnet ist. Dabei kann der Klingenträger 50 einstückig mit dem Handgriff 20 verbunden sein oder, wie in den Figuren des vorliegenden Ausführungsbeispiels dargestellt, über eine lösbare Verbindung, beispielsweise eine Schraubverbindung 60 oder eine Bajonettverbindung, lösbar an dem Handgriff 20 angeordnet sein. Dies ermöglicht ein einfaches Auswechseln der Klinge 30, indem die Klinge 30 einschließlich des Klingenträgers 50 ausgetauscht werden kann.

Das Instrument 10 kann einen ersten Abschnitt 14, welcher beispielsweise größtenteils durch den Handgriff 20 gebildet ist, mit einer ersten Längsachse 11 und einen zweiten Abschnitt 15, welcher beispielsweise größtenteils durch den Klingenträger 50 gebildet ist, mit einer zweiten Längsachse 12 aufweisen. Die Längsachsen 11 und 12 können fluchten. Vorteilhafterweise ist jedoch die zweite Längsachse 12 um einen Winkel α gegen die erste Längsachse 11 abgewinkelt, wobei der Winkel α im Bereich von 10° bis 40°, vorzugsweise im Bereich von 20° bis 30° liegt und beispielsweise 25° beträgt. Dabei liegt der Winkel α, um den die zweite Längsachse 12 gegen die erste Längsachse 11 abgewinkelt ist, insbesondere in der Ebene EK der Klinge 30 (vgl. Figur 7).

Das Instrument 10 kann stiftartig mit einem distalen Ende 11 und einem proximalen Ende 12 ausgebildet sein, wobei die Klinge 30 an dem distalen Ende 11, insbesondere stirnseitig an dem distalen Ende 11, angeordnet ist. Dabei ist die Klinge 30 insbesondere derart ausgerichtet, dass die Längsachse 12 des ersten Abschnitts 14, insbesondere des Handgriffs 20, in der Ebene der Klinge 30 liegt.

Das chirurgische Instrument 10 weist ultraschallsichtbarkeitsverstärkende Mittel 40 auf. Diese können im Bereich des distalen Endes 11 oder in der Nähe dazu angeordnet sein und sind vorzugsweise an der Klinge 30 angeordnet. Die Mittel 40 können unlösbar an der Klinge 30 oder dem Klingenträger 50 angeordnet sein. Dazu können die Mittel 40 beispielsweise angeklebt oder einstückig mit der Klinge 30 oder dem Klingenträger 50 verbunden sein. Die Mittel 40 können auch angeclippt oder aufgerastet werden. Die Mittel 40 können mit der Klinge 30 oder dem Klingenträger 50 zumindest abschnittsweise vergossen sein, um Spalte oder Ritzen zu verringern oder zu vermeiden.

Die Mittel 40 umfassen insbesondere Ultraschallreflektoren, welche als Vertiefungen 44 ausgebildet sein können. Die Vertiefungen 44 sind insbesondere derart ausgebildet, dass sie den Ultraschall reflektieren, insbesondere unabhängig davon, aus welcher Richtung der Ultraschall auf die Vertiefungen 44 auftrifft. Dazu können die Vertiefungen beispielsweise genau drei jeweils senkrecht zueinander ausgerichtete Seitenflächen 44a, 44b, 44c aufweisen (vgl. insbesondere Figur 8). Dadurch ergibt sind eine Vertiefung 44 derart, als ob eine Ecke eines Würfels in eine Oberfläche eingedrückt worden wäre. Derartige Vertiefungen 44 reflektieren einen einfallenden Ultraschallstrahl unabhängig von der Einfallsrichtung parallel zur Einfallsrichtung zurück.

Eine erste Seitenfläche 44a der Vertiefung 44 kann mit der Ebene EB des Basiselements 42 einen Winkel von etwa 35° einschließen. Eine zweite Seitenfläche 44b und eine dritte Seitenfläche 44c können symmetrisch zu einer Längsachse lB des Basiselements 42 angeordnet sein. Beispielsweise kann die Vertiefung 44 eine dreieckige Grundfläche 44d in der Außenfläche des Basiselements 42 aufweisen, wobei die Spitze des Dreiecks zu dem distalen Ende 11 des chirurgischen Instruments weist (vgl. Figuren 4 und 8).

Die Mittel 40 umfassen vorzugsweise ein blattartiges Basiselement 42, welches eine Ebene EB aufweist, welche parallel zu einer Oberfläche des blattartigen Basiselements 42 verläuft. Die Ebene EB des Basiselements ist in Figur 4 die Papierebene und ist in Figur 5 im Schnitt erkennbar. Insbesondere verläuft die Ebene EB des Basiselements 42 im Wesentlichen senkrecht zur Ebene EK der Klinge 30. Die Vertiefungen 44 sind insbesondere in dem blattartigen Basiselement 42 angeordnet. Daher sollte das blattartige Basiselement 42 eine ausreichende Dicke aufweisen, die größer als die Tiefe der Vertiefungen 44 ist. Das Basiselement 42 kann direkt an einer der Seitenkanten der blattartigen Klinge 30 angeordnet sein. Alternativ kann das Basiselement 42, wie insbesondere in Figur 5 erkennbar, mittels eines Verbindungsbügels 46 an einer der Seitenkanten der blattartigen Klinge 30 angeordnet sein. Die Länge des Basiselements 42 kann dabei länger oder kürzer als die Länge der Klinge 30 sein oder dieser entsprechend.

Die Mittel 40 können zumindest abschnittsweise oder vollständig mit einer Beschichtung versehen sein, die insbesondere die Vertiefungen 44 ausfüllt und eine glatte Außenseite bildet, welche einfach zu reinigen ist. Die Mittel 40 können insbesondere aus Metall gefertigt sein, während die Beschichtung beispielsweise aus einem ultraschalltransparenten Material gefertigt sein kann. Der Klingenträger 50 und/oder der Handgriff 20 können aus Kunststoff, beispielsweise im spritzgussverfahren, hergestellt sein, wobei insbesondere der Klingenträger 50 und der Handgriff 20 als zwei Teile oder auch einstückig miteinander verbunden ausgebildet sein können.

### Bezugszeichenliste

- 10: Instrument
- 11: distales Ende
- 12: proximales Ende
- 14: erster Abschnitt
- 15: zweiter Abschnitt
- 20: Handgriff
- 30: Klinge
- 32: Schneidkante
- 40: ultraschallsichtbarkeitserhöhende Mittel
- 42: Basiselement
- 44: Vertiefung
- 44a: erste Seitenfläche
- 44b: zweite Seitenfläche
- 44c: dritte Seitenfläche
- 44d: Grundfläche
- 46: Verbindungsbügel
- 50: Klingenträger
- 60: Schraubverbindung
- EK: Ebene der Klinge
- EB: Ebene des Basiselements
- lB: Längsachse des Basiselements
- 11: erste Längsachse
- 12: zweite Längsachse
- α: Winkel

## Patentansprüche

1. Chirurgisches Instrument (10) mit einem Handgriff (20) und einer daran angeordneten Klinge (30) mit einer konkav ausgebildeten Schneidkante (32), wobei die Klinge (30) eine Ebene (EK) aufweist,
**dadurch gekennzeichnet, dass** das chirurgische Instrument (10) ultraschallsichtbarkeitserhöhende Mittel (40) aufweist, welche Ultraschallreflektoren umfassen, wobei die Ultraschallreflektoren als Vertiefungen (44) ausgebildet sind, welche jeweils mindestens zwei jeweils senkrecht zueinander ausgerichtete Seitenflächen (44a, 44b, 44c) aufweisen, wobei die Mittel (40) ein blattartiges Basiselement (42) aufweisen, welches eine Ebene (EB) aufweist, wobei die Ultraschallreflektoren auf dem Basiselement (EB) angeordnet sind, und wobei die Ebene (EB) des blattartigen Basiselements (42) im Wesentlichen senkrecht zu der Ebene (EK) der Klinge (30) angeordnet ist.

2. Chirurgisches Instrument nach Anspruch 1,
**dadurch gekennzeichnet, dass** die Vertiefungen (44) jeweils genau drei jeweils senkrecht zueinander ausgerichtete Seitenflächen (44a, 44b, 44c) aufweisen.

3. Chirurgisches Instrument nach einem der vorhergehenden Ansprüche,
**dadurch gekennzeichnet, dass** die Mittel (40) unlösbar an der Klinge (30) angeordnet sind, beispielsweise angeklebt sind oder einstückig mit dieser verbunden sind.

4. Chirurgisches Instrument nach einem der vorhergehenden Ansprüche,
**dadurch gekennzeichnet, dass** die Mittel (40) zumindest abschnittsweise eine Beschichtung aufweisen.

5. Chirurgisches Instrument nach einem der vorhergehenden Ansprüche,
**dadurch gekennzeichnet, dass** die Mittel (40) mit der Klinge (30) zumindest abschnittsweise vergossen sind.

6. Chirurgisches Instrument nach einem der Ansprüche 1 bis 5,
**dadurch gekennzeichnet, dass** eine erste der Seitenflächen (44a) einer der Vertiefungen (44) mit der Ebene (EB) des Basiselements (42) einen Winkel (α) von etwa 35° einschließt.

7. Chirurgisches Instrument nach einem der Ansprüche 1 bis 6,
**dadurch gekennzeichnet, dass** eine zweite und eine dritte der Seitenflächen (44b, 44c) symmetrisch zu einer Längsachse (lB) des Basiselements (42) angeordnet sind.

8. Chirurgisches Instrument nach einem der Ansprüche 1 bis 7,
**dadurch gekennzeichnet, dass** die Vertiefungen (44) eine dreieckige Grundfläche (44d) in der Außenfläche des Basiselements (42) aufweisen, wobei die Spitze des Dreiecks zu einem distalen Ende (11) des chirurgischen Instruments (10) weist.

9. Chirurgisches Instrument nach einem der vorhergehenden Ansprüche,
**dadurch gekennzeichnet, dass** das chirurgische Instrument (10) stiftartig mit einem distalen Ende (11) und einem proximalen Ende (12) ausgebildet ist, wobei die Klinge (30) an dem distalen Ende (11), vorzugsweise stirnseitig am distalen Ende (11), angeordnet ist.

10. Chirurgisches Instrument nach einem der vorhergehenden Ansprüche,
**dadurch gekennzeichnet, dass** die Klinge (30) an einem Klingenträger (50) angeordnet ist.

11. Chirurgisches Instrument nach Anspruch 10,
**dadurch gekennzeichnet, dass** die Mittel (40) unlösbar an dem Klingenträger (50) angeordnet sind, beispielsweise angeklebt sind oder einstückig mit diesem verbunden sind.

12. Chirurgisches Instrument nach Anspruch 10 oder 11,
**dadurch gekennzeichnet, dass** die Klinge (30) oder der Klingenträger (50) lösbar an dem Handgriff (20) befestigt ist, vorzugsweise mittels einer Schraubverbindung (60) oder einer Bajonettverbindung.

13. Chirurgisches Instrument nach Anspruch 10 bis 12,
**dadurch gekennzeichnet, dass** das chirurgische Instrument (10) einen ersten Abschnitt (15), welcher vorzugsweise im Wesentlichen von dem Handgriff (20) gebildet ist, mit einer ersten Längsachse (11) und einen zweiten Abschnitt (15), welcher vorzugsweise im Wesentlichen von dem Klingenträger (50) gebildet ist, mit einer zweiten Längsachse (12) aufweist, wobei die zweite Längsachse (12) um einen Winkel (α) gegen die erste Längsachse (11) abgewinkelt ist, welcher im Bereich von 10° bis 40°, vorzugsweise im Bereich von 20° bis 30°, liegt und besonders bevorzugt 25° beträgt.

14. Chirurgisches Instrument nach Anspruch 13,
**dadurch gekennzeichnet, dass** der Winkel (α), um den die zweite Längsachse (12) gegen die erste Längsachse (11) abgewinkelt ist, in der Ebene (EK) der Klinge (30) liegt.

## Claims

1. Surgical instrument (10), having a handle (20) and a blade (30) arranged thereupon which has a concavely formed cutting edge (32), wherein the blade (30) comprises a plane (EK),
**characterized in that** the surgical instrument (10) comprises means for enhancing ultrasound visibility (40) which includes ultrasound reflectors, wherein the ultrasound reflectors are implemented as indentations (44) which comprise at least two lateral surface (44a, 44b, 44c), oriented perpendicular to each other, wherein the means (40) comprise a leaf-like base element (42) which comprises a plane (EB), wherein the ultrasound reflectors are arranged on the base element (42), and wherein the plane (EB) of the leaf-like base element (42) is arranged substantially perpendicular to the plane (EK) of the blade (30).

2. Surgical instrument in accordance with claim 1,
**characterized in that** each of the indentations (44) comprises exactly three lateral surfaces (44a, 44b, 44c), each of which is arranged perpendicular to the others.

3. Surgical instrument in accordance with either of the preceding claims,
**characterized in that** the means (40) are non-detachably arranged on the blade (30), for example, are adhered or integrally connected to the latter.

4. Surgical instrument in accordance with any of the preceding claims,
**characterized in that** the means (40) comprise, at least in some sections, a coating.

5. Surgical instrument in accordance with any of the preceding claims,
**characterized in that** the means (40) are at least partially molded together with the blade (30).

6. Surgical instrument in accordance with any of claims 1 to 5,
**characterized in that** a first one of the lateral surfaces (44a) of one of the indentations (44) forms, together with the plane (EB) of the base element (42), an angle (α) of approximately 35°.

7. Surgical instrument in accordance with any of claims 1 to 6,
**characterized in that** a second and a third one of the lateral surfaces (44b, 44c) are arranged symmetrically to a longitudinal axis (1_{B}) of the base element (42).

8. Surgical instrument in accordance with any of claims 1 to 7,
**characterized in that** the indentations (44) comprise a triangular base (44d) in the outer surface of the base element (42), wherein the apex of the triangle points toward a distal end (11) of the surgical instrument (10).

9. Surgical instrument in accordance with any of the preceding claims,
**characterized in that** the surgical instrument (10) is implemented in a pen-like form, with a distal end (11) and with a proximal end (12), wherein the blade (30) is arranged on the distal end (11), preferably on the end face of the distal end (11).

10. Surgical instrument in accordance with any of the preceding claims,
**characterized in that** the blade (30) is arranged on a blade holder (50).

11. Surgical instrument in accordance with claim 10,
**characterized in that** the means (40) are non-detachably arranged on the blade holder (50), for example, are adhered or integrally connected to the latter.

12. Surgical instrument in accordance with claim 10 or 11,
**characterized in that** the blade (30) or the blade holder (50) is detachably attached to the handle (20), preferably by means of a screwed connection (60) or a bayonet connection.

13. Surgical instrument in accordance with any of claims 10 to 12,
**characterized in that** the surgical instrument (10) comprises a first section (15), which is preferably substantially formed by the handle (20) and which has a first longitudinal axis (11), and a second section (15), which is preferably substantially formed by the blade holder (50) and which has a second longitudinal axis (12), wherein the second longitudinal axis (12) is bent toward the first longitudinal axis (11) at an angle (α) which is within the range of 10° to 40°, preferably in the range of 20° to 30°, and which is particularly preferably at 25°.

14. Surgical instrument in accordance with claim 13,
**characterized in that** the angle (α) at which the second longitudinal axis (12) is bent toward the first longitudinal axis (11) is in the plane (EK) of the blade (30).

## Revendications

1. Instrument chirurgical (10) comportant une poignée (20) munie d'une lame (30) ayant une arête de coupe (32) de forme concave, la lame (30) ayant un plan (EK),
instrument chirurgical (10) **caractérisé en ce qu'**il comprend :
des moyens augmentant la visibilité aux ultrasons (40) avec des réflecteurs d'ultrasons,
- les réflecteurs d'ultrasons étant réalisés sous la forme de cavités (44) ayant au moins deux côtés (44a, 44b, 44c) perpendiculaires l'un à l'autre,
- les moyens (40) comprenant un élément de base (42) en forme de lame ayant un plan (EB),
- les réflecteurs d'ultrasons étant installés sur l'élément de base (EB), et
- le plan (EB) de l'élément de base (42) en forme de lame est pratiquement perpendiculairement au plan (EK) de la lame (30).

2. Instrument chirurgical selon la revendication 1,
**caractérisé en ce que**
les cavités (44) ont chacune exactement trois côtés (44a, 44b, 44c) orientés perpendiculairement l'un à l'autre.

3. Instrument chirurgical selon l'une des revendications précédentes,
**caractérisé en ce que**
les moyens (40) sont non amovible sur la lame (30), par exemple, par collage ou reliés en une seule pièce à la lame.

4. Instrument chirurgical selon l'une des revendications précédentes,
**caractérisé en ce que**
les moyens (40) ont, au moins par segments, un revêtement.

5. Instrument chirurgical selon l'une des revendications précédentes,
**caractérisé en ce que**
les moyens (40) sont coulés au moins par segment avec la lame (30).

6. Instrument chirurgical selon l'une des revendications 1 à 5,
**caractérisé en ce que**
un premier côté (44a) de l'une des cavités (44) fait avec le plan (EB) de l'élément de base (42) un angle (α) d'environ 35°.

7. Instrument chirurgical selon l'une des revendications 1 à 6,
**caractérisé en ce que**
un second et un troisième côtés (44b, 44c) sont symétriques par rapport à l'axe longitudinal (1B) de l'élément de base (42).

8. Instrument chirurgical selon l'une des revendications 1 à 7,
**caractérisé en ce que**
les cavités (44) ont une surface de base triangulaire (44d) dans la surface extérieure de l'élément de base (42),
la pointe du triangle étant tournée vers l'extrémité distale (11) de l'instrument chirurgical (10).

9. Instrument chirurgical selon l'une des revendications précédentes, **caractérisé en ce que**
l'instrument chirurgical (10) est en forme de tige avec une extrémité distale (11) et une extrémité proximale (12),
- la lame (30) étant sur l'extrémité distale (11), de préférence sur le côté frontal de l'extrémité distale (11).

10. Instrument chirurgical selon l'une des revendications précédentes,
**caractérisé en ce que**
la lame (30) est sur un porte-lame (50).

11. Instrument chirurgical selon la revendication 10,
**caractérisé en ce que**
les moyens (40) sont non détachables sur le porte-lame (50), par exemple, ils sont collés ou reliés en une seule pièce avec celui-ci.

12. Instrument chirurgical selon la revendication 10 ou 11,
**caractérisé en ce que**
la lame (30) ou le porte-lame (50) est fixé de manière amovible à la poignée (20), de préférence par une liaison à vis (60) ou une liaison de type baïonnette.

13. Instrument chirurgical selon les revendications 10 à 12,
**caractérisé en ce que**
l'instrument chirurgical (10) a un premier segment (15) qui est de préférence constitué pratiquement par la poignée (20), avec un premier axe longitudinal (11) et un second segment (15) qui est de préférence formé essentiellement par le porte-lame (50), ayant un second axe longitudinal (12),
le second axe longitudinal (12) faisant un angle (α) par rapport au premier axe longitudinal (11) cet angle étant dans une plage comprise entre 10° et 40°, de préférence dans une plage comprise entre 20° et 30° et d'une manière particulièrement préférentielle, l'angle est égal à 25°.

14. Instrument chirurgical selon la revendication 13,
**caractérisé en ce que**
l'angle (α) entre le second axe longitudinal (12) et le premier axe longitudinal (11) est dans le plan (EK) de la lame (30).
